# EUROPEAN PATENT APPLICATION

(11) **EP 1 249 503 A2**
(43) Date of publication of application: **16.10.2002**
(21) Application number: 02076409.8
(22) Date of filing: 11.04.2002
(51) Int. Cl.: C12Q 1/68

(54) **Multiplexed gene analysis on a mobile solid support**

(30) Priority: 13.04.2001 US 834470
(71) Applicant: SMITHKLINE BEECHAM CORPORATION, Philadelphia Pennsylvania 19103 (US)
(72) Inventor: Chen, Jingwen, c/o GlaxoSmithKline, Research Triangle Park, NC 27709 (US); Taylor, David D., c/o GlaxoSmithKline, Research Triangle Park, NC 27709 (US); Weiner, Michael Philip, c/o GlaxoSmithKline, Research Triangle Park, NC 27709 (US); Ye, Fei, c/o GlaxoSmithKline, Research Triangle Park, NC 27709 (US)
(74) Representative: Giddings, Peter John, Dr.

(57) **Abstract**

The present invention relates to methods and kits for identifying and quantifying target nucleic acids in an array of assay samples using distinguishably labeled microspheres and a detecting means, such as flow cytometry. The methods and kits can be used in a multiplexed format to provide a highly sensitive, low cost, high-throughput assay of many targets simultaneously without the spatial constraints of a fixed array

## Description

### BACKGROUND OF THE INVENTION

### FIELD OF THE INVENTION

This invention relates generally to methods for rapid detection and quantification of target nucleic acids in an array of assay samples using labeled microspheres as the detection platform. The invention can be utilized to perform differential gene expression assays, such as the nuclease protection assay, with a microsphere detection platform in order to screen or quantify large numbers of nucleic acids in a high-throughput multiplexed assay.

### BACKGROUND ART

Differential gene expression provides a means for analyzing the link between specific genes and phenotypic expression and for analyzing the effects of drugs or other treatments on gene expression. Traditionally, differential gene expression studies have necessitated sequencing the gene or genes to be studied and expressing those genes in a cell line. These studies were based on enumerating the occurrence of sequences representing each transcript in libraries prepared from a specific cell or tissue. Such sequenced-based studies, which are costly and laborious, yield a limited amount of information about expression patterns.

Array-based approaches, called DNA microarrays or DNA chip arrays, have been introduced to allow for screening of large set of genes simultaneously. In these approaches, a large set of genes, in some cases the complete set of recognized genes in a genome, is represented by a dense, compact, ordered array of oligonucleotides or PCR products on a solid substrate like a microscope slide. In this fixed array, each spot on the slide represents a specific gene. Fluorescently labeled cDNAs derived from mRNAs from a sample are hybridized to these spots to determine which genes are expressed in that sample. The fluorescence associated with each gene can then be quantified.

More recently, multi-array plate screening (MAPS) technology was introduced by Siddco. MAPS assays are based on an array that is repeated in each well of a microplate and used in conjunction with a nuclease protection assay. Partial gene sequences or expression sequence tags can be used with a MAPS assay, thus negating the need for fully characterized gene sequences or promoters. Like the DNA chip arrays, each element of the array binds a different target molecule and forms a spatial pattern. Also like the DNA chip array, the number of targets per well that can be analyzed is limited by the spatial constraints of the fixed solid support.

Needed in the art was an arrayed platform that provides a sensitive, low cost, high-throughput assay of many targets simultaneously without the spatial constraints of a fixed array. Also needed in the art were internal standards for each well. Sensitivity of a nuclease protection assay, for example, depends upon complete digestion of DNA probes not hybridized to the RNA target. Prior to the present invention, a negative control to determine assay background values was performed by setting up a duplicate reaction containing all reaction components except for the target RNA. A positive control was performed in a third reaction containing all components except for the nuclease and target RNA. These controls had the advantage of using the protection probe for the gene of interest, thereby controlling for any sequence-specific variation in hybridization efficiency or nuclease digestion. However, these types of controls had disadvantages because of demanding a three-fold increase in the number of reactions assembled and because of their assumption that reaction progress in the control wells is identical to that in the separate sample well.

### SUMMARY OF THE INVENTION

In accordance with the purposes of this invention, as embodied and broadly described herein, this invention, in one aspect, relates to methods of identifying and quantifying target nucleic acids in an array of assay samples. In one embodiment, the invention relates to an identification method comprising (a) performing an arrayed nuclease protection assay to produce one or more assay products in each sample, wherein each assay product is specific for a target nucleic acid, wherein each assay product comprises a series of hybridized nucleic acid sequences, wherein one of the nucleic acid sequences is detectably tagged and wherein one of the nucleic acid sequences is attached to a distinguishably labeled microsphere; and (b)detecting with a detecting means the detectable tag and the distinguishable label in the assay products of each sample to identify the presence of a specific assay product. The presence of specific assay products in the specific arrayed samples identifies the target nucleic acids. The present method is performed in the presence or absence of standards in each arrayed sample.

In another aspect, the invention relates to a quantification method comprising the steps of (a) performing an arrayed nuclease protection assay to produce one or more assay products in each sample, wherein each assay product is specific for a target nucleic acid, wherein each assay product comprises a series of hybridized nucleic acid sequences, wherein one of the nucleic acid sequences is detectably tagged and wherein one of the nucleic acid sequences is attached to a distinguishably labeled microsphere; (b) detecting with a detecting means the detectable tag and the distinguishable label in the assay products of each sample to identify the presence of a specific assay product; and (c) quantifying the amount of detectable tag associated with each distinguishable label. The amount of detectable tag associated with each distinguishable label in the specific arrayed samples quantifies the specific target nucleic acids. Optionally, internal standards are included in each arrayed sample.

In yet another aspect, the invention relates to an identification method comprising the steps of (a) contacting one or more protection probes with one or more target nucleic acids in the array of samples under conditions that allow protection probes complementary to the target nucleic acids to hybridize with the target nucleic acids to form first hybridization products, wherein each first hybridization product comprises a protection probe and a target nucleic acid, and wherein each protection probe comprises a detectable tag and a nucleic acid sequence complementary to one target nucleic acid to be identified in one or more specific samples in the array; (b) contacting the arrayed samples with a nuclease that cleaves non-hybridized nucleic acids, under conditions that allow the first hybridization products to be protected from the cleavage; (c) contacting the protection probes of the first hybridization products with one or more linker probes and one or more anchor probes under conditions that allow formation of one or more second hybridization products, wherein the second hybridization products comprise a protection probe, a linker probe, and an anchor probe, wherein each linker probe comprises a sequence that is capable of hybridizing with one protection probe and a sequence that is capable of hybridizing with one anchor probe in the sample, and wherein each anchor probe is coupled with a microsphere with a distinguishable label; and (d) detecting the presence of the detectable tag of the protection probe and the label of the microsphere in each second hybridization product in each sample. The presence of the detectable tag and the label of the microsphere in a specific sample in the array identifies the target nucleic acids. The use of internal stands can optionally be included with each specific sample.

The invention also relates to a quantification method comprising the steps of (a) contacting one or more protection probes with one or more target nucleic acids in the array of samples under conditions that allow protection probes complementary to the target nucleic acids to hybridize with the target nucleic acids to form first hybridization products, wherein each first hybridization product comprises a protection probe and a target nucleic acid, and wherein each protection probe comprises a detectable tag and a nucleic acid sequence complementary to one target nucleic acid to be identified in one or more specific samples in the array; (b) contacting the arrayed samples with a nuclease that cleaves non-hybridized nucleic acids, under conditions that allow the first hybridization products to be protected from the cleavage; (c) contacting the protection probes of the first hybridization products with one or more linker probes and one or more anchor probes under conditions that allow formation of one or more second hybridization products, wherein the second hybridization products comprise a protection probe, a linker probe, and an anchor probe, wherein each linker probe comprises a sequence that is capable of hybridizing with one protection probe and a sequence that is capable of hybridizing with one anchor probe in the sample, and wherein each anchor probe is coupled with a microsphere with a distinguishable label; (d) detecting the presence of the detectable tag of the protection probe and the label of the microsphere in each second hybridization product in each sample; and (e) quantifying the amount of detectable tag associated with each distinguishable label. The amount of detectable tag associated with each distinguishable label in the specific arrayed samples is used to quantify the target nucleic acids. The use of internal standards can optionally be included with each specific sample. The present invention further relates to a kit for performing the methods described herein. Specifically, the invention provides a kit for identifying or quantifying target nucleic acids in an array of assay samples, comprising a set of microspheres coupled with a set of anchor probes, wherein the set of microspheres comprises subsets of microspheres having distinguishable labels, wherein the set of anchor probes comprises a subset of probes, wherein each subset of anchor probes is coupled with only one subset of microspheres, and wherein each subset of anchor probes is capable of hybridizing under hybridizing conditions to a subset of linker probes specific for a target nucleic acid to be quantified. The kit of the invention optionally includes internal stands for each sample in the array. The methods and kits of the present invention provide an arrayed platform that provides a highly sensitive, low cost, high-throughput assay of many targets simultaneously without the spatial constraints of a fixed array. Additional advantages of the invention will be set forth in part in the description that follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this specification, illustrate (one) several embodiment(s) of the invention and together with the description, serve to explain the principles of the invention.

Figure 1 shows an assay product of a five-oligo multi-array bead screening assay.

Figure 2 shows an assay product of a four-oligo multi-array bead screening assay.

Figure 3 shows an assay product of a three-oligo multi-array bead screening assay.

Figure 4 shows the difference in senstivity using five-oligo, four-oligo, and three-oligo multi-array bead screening systems with target nucleic acid quantities varying from 0 to 2 fmol. At concentrations of 0.002 fmol and higher of target nucleic acid, the three-oligo system provided substantially greater signal as compared to the 4-oligo and 5-oligo systems.

Figure 5 shows the results of a three-oligomulti-array assay using total human liver RNA (0, 2, and 10 µg of total RNA) and protection probes specific for the housekeeping genes GAPDH and β-actin.

Figure 6 shows the results of titration of 0.2-200 fmol of linker and 0.2-2 fmols of target in a 3-oligo system.

### DESCRIPTION OF THE PREFERRED EMBODIMENTS

The present invention provides a method of identifying or quantifying target nucleic acids using an array of assay samples and a mobile solid support for a detection platform. Such methods are useful in diagnostic tests and in tests for determining the efficacy of drugs or other treatments where high-throughput analysis of target nucleic acids is desired.

Before the present compounds, compositions, articles, devices, and/or methods are disclosed and described, it is to be understood that this invention is not limited to specific methods, specific nucleic acids, or to particular means of performing or using the specific methods, as such may, of course, vary. It is also to be understood that the terminology used herein is for the purpose of describing particular embodiments only and is not intended to be limiting.

The present invention may be understood more readily by reference to the following detailed description of preferred embodiments of the invention and the Examples included therein and to the Figures and their previous and following description.

Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

In this specification and in the claims that follow, reference will be made to a number of terms which shall be defined to have the following meanings:
"Optional" or "optionally" means that the subsequently described event or circumstance may or may not occur, and that the description includes instances where said event or circumstance occurs and instances where it does not.

As used in the specification and the appended claims, the singular forms "a," "an" and "the" include plural referents unless the context clearly dictates otherwise. Thus, for example, reference to "an array of assay samples" includes one or more arrays of assay samples and "a nucleic acid" includes mixtures of two or more such nucleic acids, and the like.

Specifically, the present invention provides a method of identifying target nucleic acids in an array of assay samples, comprising (a) performing an arrayed nuclease protection assay to produce one or more assay products in each sample, wherein each assay product is specific for a target nucleic acid, wherein each assay product comprises a series of hybridized nucleic acid sequences, wherein one of the nucleic acid sequences is detectably tagged and wherein one of the nucleic acid sequences is attached to a distinguishably labeled microsphere; and (b)detecting with a detecting means the detectable tag and the distinguishable label in the assay products of each sample to identify the presence of a specific assay product. The presence of specific assay products in the specific arrayed samples identifies the target nucleic acids.

In one embodiment, the invention provides a method of quantifying target nucleic acids in an array of assay samples. Specifically, the method comprises the steps of (a) performing an arrayed nuclease protection assay to produce one or more assay products in each sample, wherein each assay product is specific for a target nucleic acid, wherein each assay product comprises a series of hybridized nucleic acid sequences, wherein one of the nucleic acid sequences is detectably tagged and wherein one of the nucleic acid sequences is attached to a distinguishably labeled microsphere; (b) detecting with a detecting means the detectable tag and the distinguishable label in the assay products of each sample to identify the presence of a specific assay product; and (c) quantifying the amount of detectable tag associated with each distinguishable label. The amount of detectable tag associated with each distinguishable label in the specific arrayed samples quantifies the specific target nucleic acids.

As used throughout, an "array" includes one or more multiwell arraying means such as microplates or slides. Preferably, the array can be read using an automated reader. In preferred embodiments, the array is a microplate having 96-wells, 384-wells, or 1536-wells or groups of the same. Thus, the array could comprise 100 96-well plates.

As used throughout, "target nucleic acids" in the samples are selected from the group consisting of genomic DNA, amplified DNA (such as a PCR product), cDNA, mRNA, cRNA, a restriction, an oligonucleotide, 16s ribosomal RNA, DNA fragment, an RNA molecule, a LCR (ligase chain reaction) product, or any other desired nucleic acid. In a preferred embodiment, the target nucleic acids are mRNAs.

When genomic DNA comprises the target nucleic acids, the genomic DNA will be treated in a manner to reduce viscosity of the DNA and allow better contact of a primer or probe with the target region of the genomic DNA. Such reduction in viscosity can be achieved by any desired method, which are known to the skilled artisan, such as DNase treatment or shearing of the genomic DNA, preferably lightly. Sources of genomic DNA are numerous and depend upon the purpose of performing the methods, but include any tissue, organ or cell of choice.

Amplified DNA can be obtained by any of several known methods. Oligonucleotides can be generated by amplification or by de novo synthesis, for example. Complementary nucleic acids, *i.e.,* cRNA (obtained from a process wherein DNA is primed with a T7-RNA polymerase/specific sequence primer fusion, then T7 RNA polymerase is added to amplify the first strand to create cRNA) and cDNA, can be obtained by standard methods known in the art

As used throughout, a "mobile solid support" refers to a set of distinguishably labeled microspheres or beads. Preferably, the microspheres are polystyrenedivinylbenzene beads. Sets of microspheres marked with specific fluorescent dyes and having specific fluorescent profiles can be obtained commercially, for example, from Luminex Corporation (Austin, TX). As used throughout, a "detecting means" includes any methods of differentiating the distinguishable labels and detectable tags, including, for example, flow cytometry or confocal miscroscopy analysis. Flow cytometry can used to read the distinguishable labels and detectable tags (See WO99/36564, which is incorporated herein in its entirety for methods of flow cytometry). For purposes of confocal microscopy, the beads are placed on a slide and the detectable tags and distinguishable labels differentiated.

As used in the various embodiments of the invention, an anchor probe is coupled, directly or indirectly, to the mobile solid support. "Coupled directly or indirectly" will be understood by one skilled in the art to include various methods for coupling. For example, the microspheres can be coated with streptavidin or maleimide or can be carboxylated or amidated, or any modification of streptavidin, maleimide, carboxylation, or amidation and the anchor probes to be coupled to these microspheres can be biotintylated, have one or more free amine or carboxyl groups, or have one or more free sulfhydryl groups. One skilled in the art would recognize that other coupling agents can be used. See, e.g., WO 99/19515 and WO 99/37814, which are incorporated herein by reference in their entirety for types of functional groups that can be used for coupling the anchor probes to the microspheres. Optionally, a linker can be used between the microsphere and the coupling agent. More specifically, the anchor probe can be indirectly coupled to the mobile solid support by a carbon spacer. The anchor probe can coupled at either its 5' or 3' end to the mobile solid support.

In one embodiment, the bead is streptavidin-coated, and the anchor probe is biotinylated, and thereby the biotin on the anchor probe and the streptavidin on the bead providing a high affinity binding between the anchor probe and the bead. One skilled in the art would recognize that, when biotin is used as a means of labeling the protection probe and as a means of binding the anchor probe to the mobile solid support, the streptavidin on the mobile solid support must be saturated with biotin to prevent direct binding of the biotin of the protection probe to the mobile solid support.

One microsphere preferably has only one anchor probe coupled to it, but a plurality of the same anchor probes is preferably coupled to a single microsphere. By "a set of microspheres" is meant a group of microspheres consisting of subsets of microspheres labeled with distinguishable labels. By coupling each subset to a specific anchor probe, or a plurality of the same anchor probes, a specific label for each anchor probe can be detected.

The distinguishable labels located on or in the microspheres include dyes, radiolabels, magnetic tags, or a Quantum Dot® (Quantum Dot Corp.). In a preferred embodiment the distinguishable labels are fluorescent labels, which are contained in the microspheres and which can be detected using flow cytometry. It is well known in the art that microspheres can be labeled with two or more flurochromes mixed together in varying concentrations, such that each specific label has a specific concentration of each fluorochrome. It is the specific concentrations of the various flurochromes together to provide a spectrum of labels that can be used to distinguish the various subsets of labeled microspheres.

As used throughout, the quantification steps of the invention typically involves comparison of the amount of detectable tag to a standard or some other reference. A standard, such as that of a known amount or from a normal subject, or a diseased/ afflicted subject, or a particular tissue or organ, or a particular species, can be used as a comparison reference to draw conclusion regarding the quantity detected in the sample. A known amount of coupled microspheres is added to each assay process. The distinguishable label for uncoupled microspheres is detectable and constant for each set. Thus, it is the relative amount of detectable tag that is relevant for quantification.

As used in any of the methods described herein, a sample can be, for example, any body sample that contains nucleic acids, such as organ, tissue, and/or cells. The cells are selected, for example, from blood, red or white blood cells, bone marrow cells, neural or neuronal cells, precursor or stem cells, or cells from, liver, kidney, brain, skin, heart, lung, spleen, pancreas, gall bladder, muscle, ovaries, testicles, uterus, glands.

The arrayed nuclease protection assay used in the present invention is a modified nuclease protection assay performed according to the examples below. Specifically, the assay produces one or more assay products in each sample, wherein each assay product is specific for a target nucleic acid, wherein each assay product comprises a series of hybridized nucleic acid sequences, wherein one of the nucleic acid sequences is detectably tagged and wherein one of the nucleic acid sequences is attached to a distinguishably labeled microsphere. Preferably, the assay product comprises a series of less than five nucleic acid sequences. For example, the assay product in one embodiment comprises four nucleic acid sequences, and in another embodiment, the assay product comprises three nucleic acid sequences.

The assay is preferably a multiplexed assay in which simultaneous, or near simultaneous, determinations of binding events can be measured from the same assay process in a single well of the arrayed assay. Preferably, this multiplexed format allows a washless format, which improves sensitivity, saves reagents, and promotes efficiency.

One skilled in the art would recognize that to obtain the assay products or hybridization products according to the methods of the invention, the nucleic acid sequences must be combined under conditions that allow hybridization of complementary nucleic acid sequences. Such conditions do not require that all nucleic acids hybridize, only that the conditions allow for specific hybridization to occur. These conditions include, for example, pH, time, temperature, and buffer composition that allows specific hybridization.

Typically, the assay products comprise a distinguishably labeled microsphere coupled directly or indirectly to an oligonucleotide (referred to herein as the anchor probe) which includes a region referred to herein as a cZipCode. Preferably, the cZipCode comprises a sequence not present in a cell that contains the target nucleic acid of interest to avoid non-specific binding. The assay product further comprises a linker probe that includes a first region that is complementary to the cZipCode and a second region complementary to a specific protection probe. The protection probe comprises a region complementary to a specific target nucleic acid. Optionally, the protection probe may be directly coupled with a detectable tag (for an assay product comprising three hybridized nucleic acids) or may contain a region complementary to another linker that is coupled to a detectable tag (for an assay product comprising four hybridized nucleic acids). In one embodiment, the assay product comprises a detectably labeled protection probe. Furthermore, the assay product preferably comprises a series of less than five nucleic acid sequences. Preferably, the assay product comprises four nucleic acid sequences, and more preferably, the assay product comprises three nucleic acid sequences.

The cZipCodes and anchor probes allow the use and reuse of a defined set of optimally coupled microspheres. The target nucleic acids that can be identified in a single sample of the array is limited by the number of different labeled microspheres that can be distinguished by flow cytometry. To reduce cost and complexity, the same detectable tag can be associated with each labeled nucleic acid, or, alternatively, different detectable tags can be used in a single sample or in different samples. To further reduce cost and complexity, a comparable set of microspheres with comparable anchor probes can be used for each sample in the array. Different sets of assay products are indentified or quantified in each sample, based on the specific protection probes used in the nuclease protection assay in a given sample, to allow identification and quantification of various target nucleic acids in each sample. In one embodiment of the invention, each sample in the array contains a different set of protection probes. Optionally, the same set of distinguishably labeled microspheres is present in each sample of the array. By "the same set of microspheres" is meant a comparable set having the same distinguishable labels and the same anchor probes attached to microspheres having a specific distinguishable label.

The detectable tag of the assay product can be selected from haptens, dyes, radiolabels, magnetic tags, or a Quantum Dot® (Quantum Dot Corp.). In a preferred embodiment, the detectable tag is biotin. Biotin is detected by any one of several techniques known in the art. For example, the biotin is detectable by binding with a fluorescence-labeled avidin and the avidin is labeled with a phycoerythrin or a catenated fluorescent label to increase the signal associate with each binding event on each microsphere.

The present invention also provides a method of identifying target nucleic acids in an array of assay samples, comprising the steps of (a) contacting one or more protection probes with one or more target nucleic acids in the array of samples under conditions that allow protection probes complementary to the target nucleic acids to hybridize with the target nucleic acids to form first hybridization products, wherein each first hybridization product comprises a protection probe and a target nucleic acid, and wherein each protection probe comprises a detectable tag and a nucleic acid sequence complementary to one target nucleic acid to be identified in one or more specific samples in the array; (b) contacting the arrayed samples with a nuclease that cleaves non-hybridized nucleic acids, under conditions that allow the first hybridization products to be protected from the cleavage; (c) contacting the protection probes of the first hybridization products with one or more linker probes and one or more anchor probes under conditions that allow formation of one or more second hybridization products, wherein the second hybridization products comprise a protection probe, a linker probe, and an anchor probe, wherein each linker probe comprises a sequence that is capable of hybridizing with one protection probe and a sequence that is capable of hybridizing with one anchor probe in the sample, and wherein each anchor probe is coupled with a microsphere with a distinguishable label; and (d) detecting the presence of the detectable tag of the protection probe and the label of the microsphere in each second hybridization product in each sample. The presence of the detectable tag and the label of the microsphere in a specific sample in the array identifies the target nucleic acids.

The invention also provides a method of quantifying target nucleic acids in an array of assay samples, comprising the steps of (a) contacting one or more protection probes with one or more target nucleic acids in the array of samples under conditions that allow protection probes complementary to the target nucleic acids to hybridize with the target nucleic acids to form first hybridization products, wherein each first hybridization product comprises a protection probe and a target nucleic acid, and wherein each protection probe comprises a detectable tag and a nucleic acid sequence complementary to one target nucleic acid to be identified in one or more specific samples in the array; (b) contacting the arrayed samples with a nuclease that cleaves non-hybridized nucleic acids, under conditions that allow the first hybridization products to be protected from the cleavage; (c) contacting the protection probes of the first hybridization products with one or more linker probes and one or more anchor probes under conditions that allow formation of one or more second hybridization products, wherein the second hybridization products comprise a protection probe, a linker probe, and an anchor probe, wherein each linker probe comprises a sequence that is capable of hybridizing with one protection probe and a sequence that is capable of hybridizing with one anchor probe in the sample, and wherein each anchor probe is coupled with a microsphere with a distinguishable label; (d) detecting the presence of the detectable tag of the protection probe and the label of the microsphere in each second hybridization product in each sample; and (e) quantifying the amount of detectable tag associated with each distinguishable label. The amount of detectable tag associated with each distinguishable label in the specific arrayed samples is used to quantify the target nucleic acids.

When the second hybridization products comprise a detectably labeled protection probe, a linker probe, and an anchor probe, the linker probe optionally further comprises a spacer sequence between the sequence that is capable of hybridizing with the protection probe and the sequence capable of hybridizing with the anchor probe.

Preferably, the protection probes are less than 75 bases in length. More preferably, the protection probes are less than 50 bases in length, and, even more preferably, the protection probes are less that 40. The protection probes can be 74, 70, 65, 60, 55, 50, 45, 40, 35, 30, 25, or 20 bases in length or any number in between.

The detectable tag can be attached to either the 5' or 3' end of the protection probe and either the 5' or 3' end of the anchor probe can be coupled to the microsphere. When the second hybridization products comprise a detectably labeled protection probe, a linker probe, and an anchor probe, however, if the 5' end of the anchor probe is coupled to the microsphere then the 3' end of the protection probe must be coupled to the detectable tag. If the 3' end of the anchor probe is coupled to the microsphere then the 5' end of the protection probe must be coupled to the detectable tag.

The amount of linker probe used in each assay is highly relevant for maximizing signal intensity. If the amount of linker probe exceeds the number of sites for hybridizing to either the anchor or protection probe, then the excess linker probe competes with formation of the second hybridization products comprising a protection probe, a linker probe, and an anchor probe. Preferably only about 25% to about 75%, or any amount in between, of the anchor probes are bound by linker probe, so that the beads are undersaturated with bound linker probe. This undersaturation promotes formation of second hybridization products including all three components rather than formation of products consisting of linker probes and protection probes.

In all of the methods of the invention, internal standards can optionally be used. More preferably, one or more positive control and one or more negative controls are included in each reaction well or arrayed sample. Inclusion of the positive internal standard shows on a well-to-well or sample-to-sample basis that all system components are functioning and gives an indication of variability across the plate. Inclusion of the negative control provides a measure of the completeness of the S1 nuclease digestion in each well. If digestion is incomplete, the negative control is positive. These controls allow one skilled in the art to monitor the assay and make improvements in the protocol. Furthermore, these controls provide a means for routine assay validation. The controls can be selected from the group consisting of integrase, GFP, E4BP4, kv4.2 and Ca receptor. Even more preferably, the controls are selected from the group consisting of integrase, GFP and E4BP4. In one embodiment, the HIV integrase transcript and probe set is used as the standard internal positive control. In another embodiment, the green fluorescent protein transcript and probe set are used as the standard internal negative control. In a third embodiment, a three-component control mix containing integrase RNA transcript (about 0.25 to 2.5 nanograms (1 to 10 femtomoles), and, more preferably about 1 nanogram (8 femtomoles)), integrase protection probe (about100 to 200 femtomoles, and more preferably about 200 femtomoles), and GFP protection probe (about 100 to 200 femtomoles, and more preferably about 200 femtomoles) is used. The controls can be contained in plasmid constructs containing the T7 RNA polymerase promoter driving expression of a downstream gene, including, for example, pT7-IN containing the HIV-1 integrase gene, pVAX1-EGFP containing jellyfish enhanced green fluorescent protein, pcDNA3-ratKv4.2 containing rat brain potassium channel kv4.2, pT7:GST:linker:E4BP4 containing human E4BP4, and pcDNA3-rat-calcium receptor, and the control nucleic acids produced as taught in the Examples below. The present invention further provides a kit for performing the methods described herein. Specifically, the invention provides a kit for identifying or quantifying target nucleic acids in an array of assay samples, comprising a set of microspheres coupled with a set of anchor probes, wherein the set of microspheres comprises subsets of microspheres having distinguishable labels, wherein the set of anchor probes comprises a subset of probes, wherein each subset of anchor probes is coupled with only one subset of microspheres, and wherein each subset of anchor probes is capable of hybridizing under hybridizing conditions to a subset of linker probes specific for a target nucleic acid to be quantified. Optionally, the kit can further comprise an arraying means and one or more containers. Optionally the kit can further comprise linker probes, protection probes, or a means of detectably tagging the protection probes. Optionally the kit can further comprise hybridization buffers or a nuclease for a nuclease protection assay. Optionally the kits further comprise internal standards for each assay sample.

The following examples are put forth so as to provide those of ordinary skill in the art with a complete disclosure and description of how the compounds, compositions, articles, devices and/or methods claimed herein are made and evaluated, and are intended to be purely exemplary of the invention and are not intended to limit the scope of what the inventors regard as their invention. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### Example 1

### Preparation of Microspheres

Polystyrene microspheres (5.5 µm in diameter) with a carboxylated surface and different ratios of red and orange fluorescence were purchased from the Luminex Corp. (Austin, TX). All oligonucleotides used for covalent coupling to microspheres were synthesized with a 5' amine group, a C15 or C18 spacer, and 45 nucleotides (Oligos Etc, Bethel, ME or PE Biosystems, Foster City, CA). The 20 nucleotides nearest the 5' end comprised a common sequence derived from luciferase cDNA (5'-CAG GCC AAG TAA CTT CTT CG-3') (SEQ ID NO: 59) and were used to determine coupling efficiency to the microspheres by hybridization to a complementary fluoresceinated luciferase probe. The 25 nucleotide cZipCode at the 3' end were sequences derived from the Mycobacterium tuberculosis genome. This genome was chosen because it was a bacterial genome that had a high GC content. The selected sequences have GC-contents between 56 and 72% and predicted Tm values of 61 to 68°C.

Carboxylated microspheres (2.5 X 106 microspheres in 62 µl 0.1 M 2-[N-morpholino] ethanesulfonic acid (MES) (Sigma, St. Louis, MO)) were combined with amine-modified oligonucleotide (5 nmoles in 25 µl 0.1 M MES). At three separate times 0.3 mg of 1-ethyl-3-(3-dimethylaminopropyl) carbodilmide hydrochloride (EDC) (Pierce, Rockford, IL) was added to the microsphere mixture; at the beginning of the incubation, and then after two 20 min periods. The reaction was occasionally mixed and sonicated during the 60-min room temperature incubation to keep the microspheres unclumped and in suspension. After coupling, the microspheres were washed in 1 ml phosphate buffered saline containing 0.02% Tween 20 (Sigma, St. Louis, MO) and then in 150 µ110 mM tris [hydroxymethyll aminomethane hydrochloride / 1 mM ethylenediamine-tetraacetic acid pH 8.0 (TE). The microspheres were resuspended in 200 µl TE for storage at 4°C.

To assess the number of oligos covalently coupled to the microspheres, hybridizations were performed using 10,000 coupled microspheres and 3 picomoles of fluoresceinated oligo complementary to the 20 nucleotides of luciferase sequence on the 5'end of each cZipCode oligo. Hybridization was conducted in 3.3X SSC for 30 minutes at 45°C following a 2 minute 96°C denaturation. Microspheres were washed with 200 µl 2X SSC containing 0.02% Tween 20, resuspended in 300 µl 2X SSC containing 0.02% Tween 20 and analyzed by flow cytometry.

The specificity of the anchor probes for specific ZipCodes sequences in the linker probe was tested. A set of 58 linker probes was designed and synthesized, wherein each linker probe contained its own unique 5' ZipCode sequence complementary to one cZipCode sequence on the anchor probe. After flow cytometric analysis, ZipCodes which hybridized to multiple types of microspheres were discarded. ZipCodes were validated for specificity of hybridization by incubating a fluoresceinated oligonucleotide (with a given ZipCode sequence) with a multiplexed set of 58 cZipCode-coupled microspheres (only one microsphere type out of the set of 58 contained a perfectly complementary sequence). Cross-hybridization (or non-hybridization) of ZipCodes was infrequent but when encountered, the sequence was removed from the selection of ZipCodes and replaced with another non-cross hybridizing sequence. Five ZipCode sequences were replaced due to cross reactivity and 2 ZipCode sequences that were at first only weakly reactive showed specific hybridization upon retesting (and were therefore retained). One completely unreactive ZipCode was discarded. A second round of hybridizations demonstrated that, under our assay conditions, each of the 58 ZipCode sequences hybridized to only one of the 58 microsphere-attached, cZipCode sequences. The optimized sequences for ZipCodes are shown in Table 1. We have found no differences in genotyping ability when an SNP was analyzed using different ZipCode sequences.

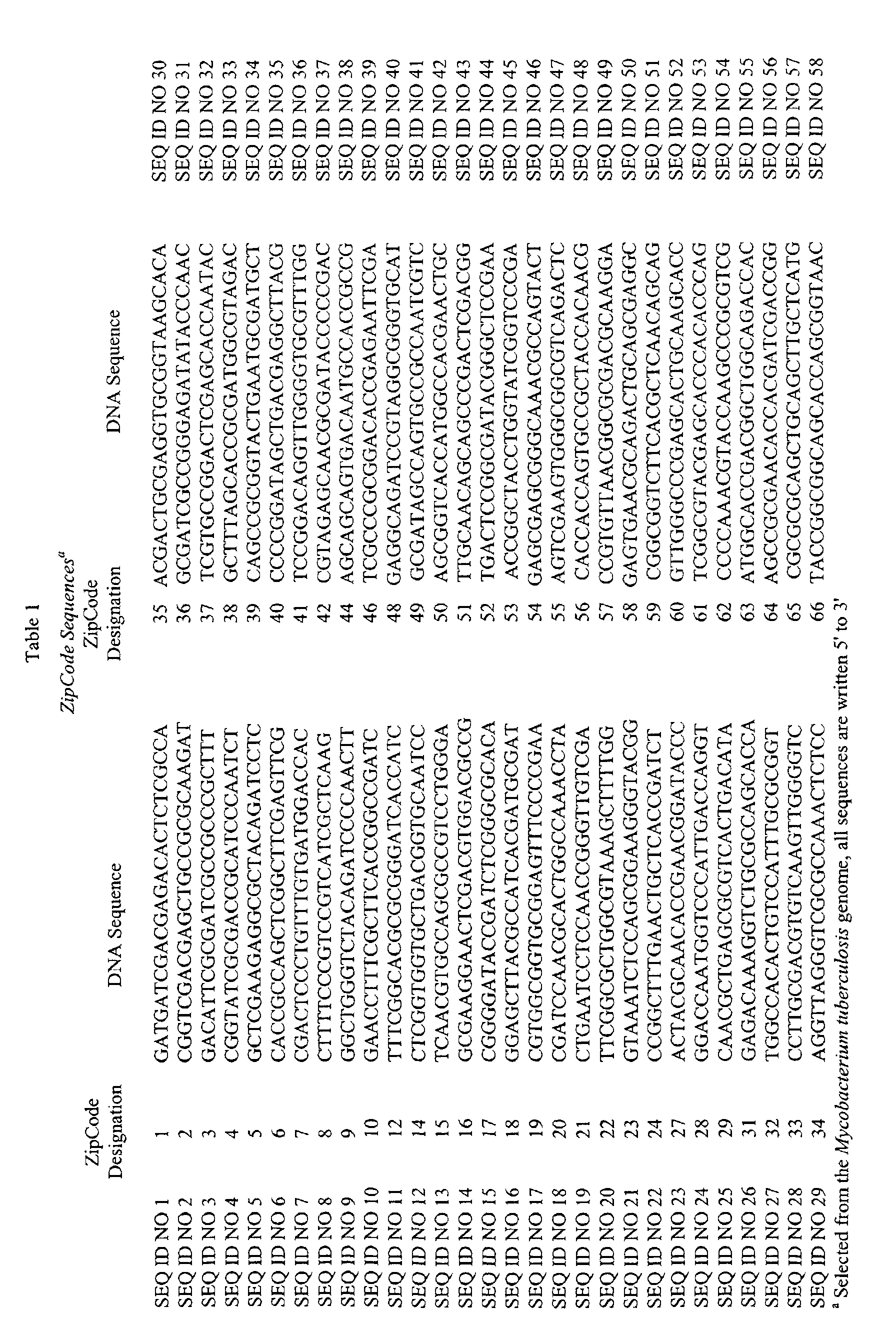

### Example 2

### Flow cytometric analysis and MESF conversions

Microsphere fluorescence was measured using a FACSCalibur flow cytometer (Becton Dickinson, San Jose, CA) equipped with Luminex Lab MAP hardware and software (Luminex Corp., Austin, TX). All green fluorescence measurements were converted to molecules of equivalent soluble fluorochrome (MESF) using Quantum Fluorescence Kit for MESF units of FITC calibration particles and QuickCal software (all obtained from Sigma, St. Louis, MO). Green fluorescence contributed by the microspheres alone were subtracted from all data points.

Conversion of raw data from mean fluorescence intensity to MESF offers several advantages. These advantages include the use of a standard fluorescence unit, the ability to compare data between experiments, the ability to compare data between instruments, and normalization of signal variability in an instrument over time (due to laser power shifts or PMT decline).

### Example 3

### Nuclease Protection Assay

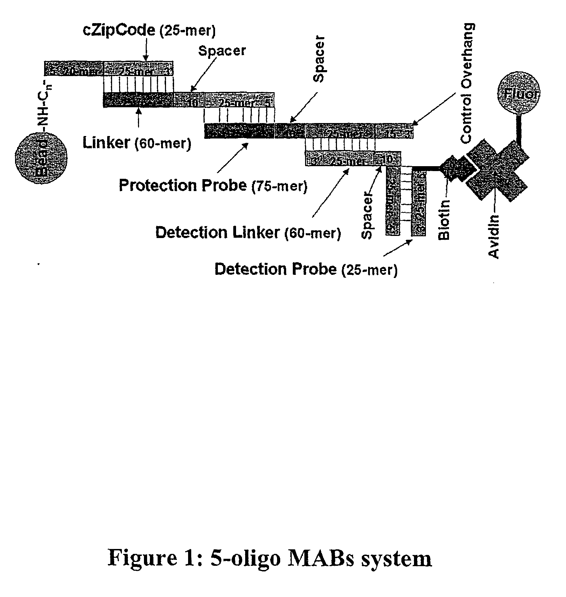

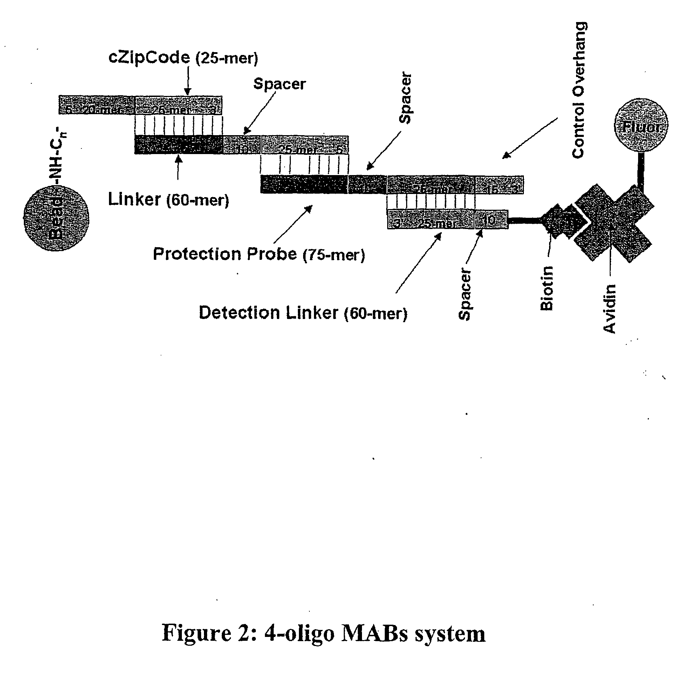

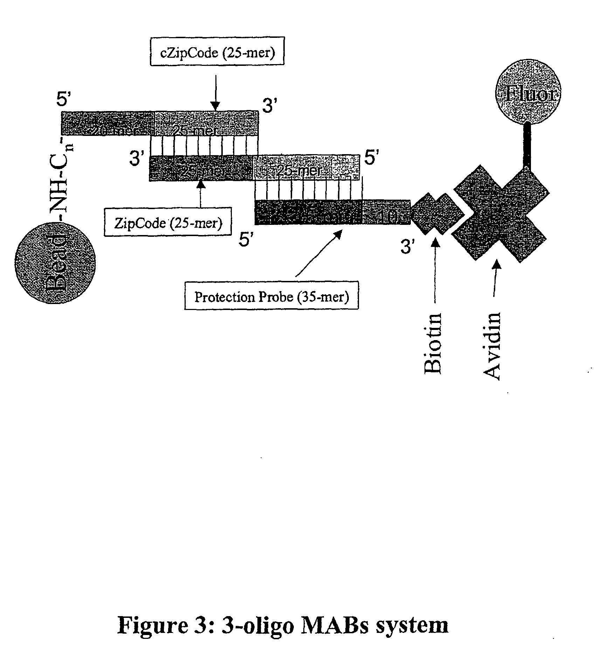

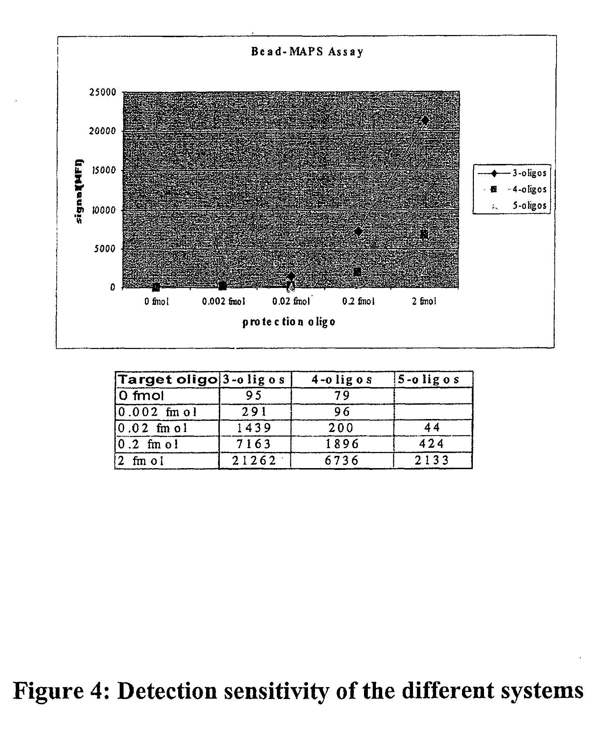

Several conditions (for example, buffers, olignucleotide concentration, and washing protocols) for the use of MAPS-derived oligonucleotide probes (SIDDCO, Phoenix, AZ) in a bead hybridization protocol were tested. The sensitivity of the bead-based differential gene expression (DGE) assay was further tested using a newly established protocol and a newly designed probe set-up. The MAPs probes tested using the bead-based assay were able to detect 0.2 fmol of a target mRNA from a cell lysate. Sensitivity of the assay using a 5-oligo system (Figure 1) was tested. To increase the assay sensitivity, both a 4-oligo and a 3-oligo system were designed and tested. (Figures 2, and 3). The results showed that the detection sensitivity can be increased by 10-100 fold (i.e., to approximately 0.02 - 0.002 fmol) when the 3-oligo system was used in the bead-based DGE assay (Figure 4).

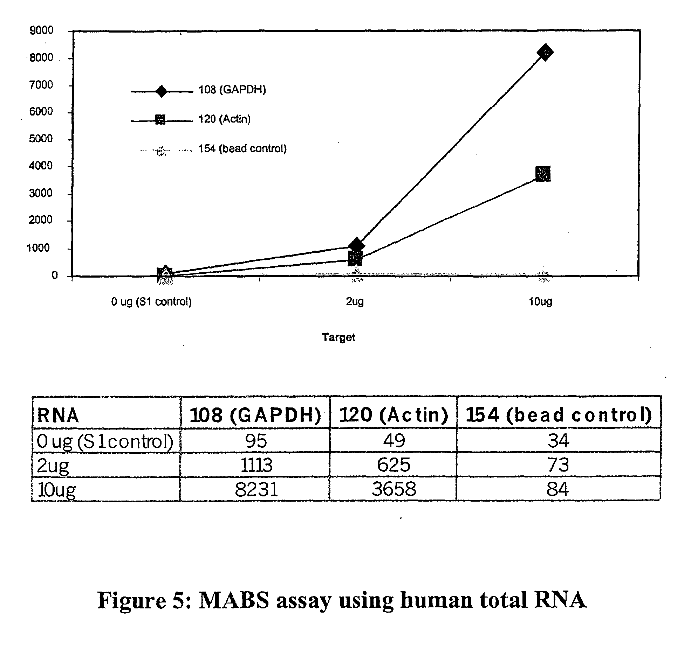

The bead-based DGE assay was also tested using total human liver RNA (S1 protection was followed by a bead-based hybridization and further signal amplification and detection). The results demonstrated that a strong signal could be obtained from 2 µg of total RNA when the housekeeping genes GAPDH and β-actin were tested under the conditions described below (Figure 5).

S1 nuclease protection. In a 96-well microtiter plate, 2-10 µg of human liver total RNA (9 µl total volume) and 1 µl of oligol (PF) mixture (20 nM for each) were added to each well. For the S control, only oligo 1 was added. After mixing, the plate was heated to 70 °C in a Tetrad PCR machine (MJ Research, Watertown, Massachusetts) for 10 min and cooled to room temperature. Subsequently, 60 µl of pre-warmed (55 °C) "Northern-Max" hybridization buffer II buffer (Ambion, Austin, Texas) was added to each well and the plate was incubated at 55°C for a further 12-16 hr.

Following hybridization, 7 µl of 10 x S1 buffer, 1 µl of S1 nuclease [50 U/µl (LTI, Gaithersburg, MD, Cat # 18001-016)] was added to each well. After incubation at 50 °C for 30 min, 10 µl of a mixture of 1 M NaOH and 10 mM EDTA was added and the plate was heated to 95 °C for 10 min, followed by incubation at room temperature for an additional 20 min. Finally, 10 µl of 1 M Tris (pH 7.2) and 3 µl of 3 M of HCl were added to neutralize the solution.

Hybridization conditions I. In a 96-well microplate, 1 µl of an oligo2 mixture (ZipLinker; 5 nM each) [ZipLinker is a bi-functional oligo (also called Linker) that includes a first region that is complementary to the cZipCode and a second region complementary to a gene-specific target nucleic acid], 1 µl of bead mixture [200 beads per each target, which were previously coupled with a cZipCode oligonucleotide, (oligo3)] and 28 µl of hybridization buffer I well (0.5 M NaCl, 13 mM EDTA, pH 8.0) were added. The mixture was incubated at 40 °C for at least 1 hr.

Following hybridization, the beads were washed three times with 120 µl of wash buffer (1 x SSC, 0.02 % Tween 20), and the plate spun in a centrifuge (SORVALL RT 5000D) at 2500 RPM for 3 min at room temperature. The supernatent was removed, leaving about 15 µl of the Wash Buffer in the well after the final wash. Optionally, instead of washing by centrifugation, a vacuum manifold with a filter plate was used to perform the washes.

Hybridization conditions II. Each of the 100 µl S1 protection product, 1 µl of oligo4 mixture (detection linker, 150 nM for each), 1 µl of 5' biotin-labeled oligo5 (detection probe, 200 nM), and 8 µl of 20X SSC were mixed with each of the hybridization I products (the final volume was adjusted to 125 µl) and incubated at 40 °C for 2 hr.

Following hybridization, the beads were washed six times with 120 µl of the wash buffer (1 x SSC, 0.02 % Tween 20) by spinning the plate in a centrifuge at 2500 RPM for 3 min at room temperature. In some cases , a vacuum manifold with a filter plate was used to perform the washes, instead of washing by centrifugation.

Signal Amplification and Detection. After the final hybridization II wash, the beads were resuspended in 65 µl a streptavidin-phycoerythrin stain solution (60 µl of wash buffer containing 5 µl of streptavidin-phycoerythrin stock at 5 µg ml-1, (Becton Dickinson, La Jolla, CA) and incubated at room temperature for 30 min. The beads were washed 6 times with 120 µl of wash buffer. After washing, 1000 µl of wash buffer containing 500 ng of biotinylated anti-streptavidin goat antibody (Vector Laboratories, Burlingame, California) (0.5 mg per ml stock in ddH2O, stored at -20 °C) were added and the solution was incubated at room temperature for 30 min in the dark. Subsequently, free conjugate was removed by washing a further 6 times with wash buffer. Beads were finally resuspended in 65 µl of the streptavidin-phycoerythrin staining solution and incubated at room temperature (in the dark) for 30 min. The plates were assayed with a LUMINEX-100 reader.

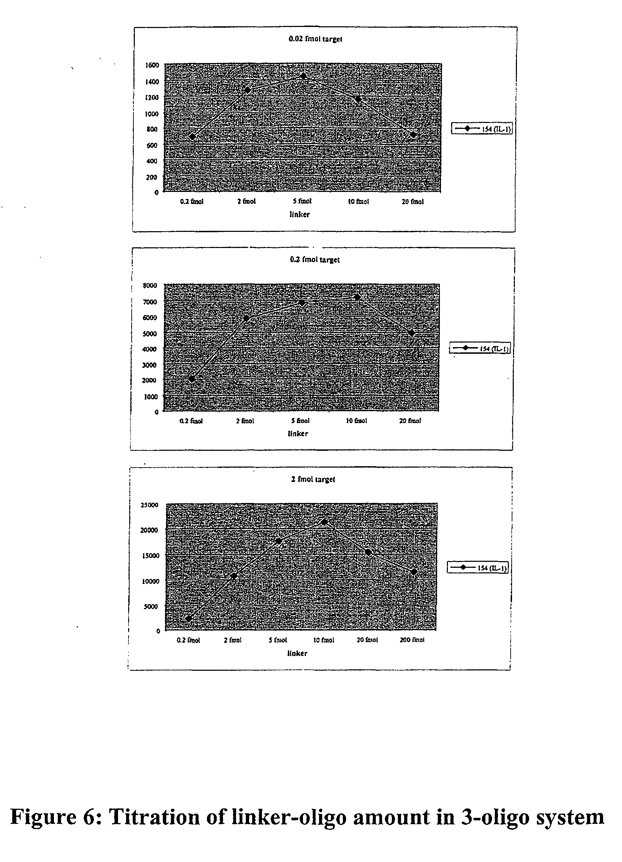

Titration of linker-oligo amount in 3-oligo system. In a 96-well microplate, 1 µl of ZipLinker mixture (Oligo 2, 0.2-20 nM for each target), 1 µl of bead mixture (200 beads per each bead type, which are coupled with cZipCode sequences, named Oligo 3) and 28 µl of Hybridization Buffer I was added to each well (Final concentration: 0.5 M NaCl, 13 mM EDTA, pH 8.0). The mixture was incubated at 40 °C for at least 1 hour. Following hybridization, the beads were washed three times with 120 µl of Wash Buffer (1 x SSC, 0.02 % Tween 20), and further hybridized with different amount of biotin-labeled protection oligo mixture (oligo 1, 0.02 - 2 fmol for each target) at 50 °C for two hours. After washing 6 times with Wash Buffer, the hybridization signals were detected through a three-step-protocol. The protocol consisted of a first incubation with a streptavidin-phycoerythrin conjugate followed by a labeling with an anti-streptavidin goat biotinylated antibody and a final staining with the streptavidin-phycoerythrin conjugate. The plates were then assayed with a Luminex-100 reader. The results are shown in Figure 6.

### Example 4

### Positive and negative controls

Candidate genes for use as positive and negative controls were selected. First, plasmids containing the T7 RNA polymerase promoter driving expression of a downstream gene were identifed. Five such plasmid constructs were selected for use: pT7-IN containing the HIV-1 integrase gene, pVAX1-EGFP containing jellyfish enhanced green fluorescent protein, pcDNA3-ratKv4.2 containing rat brain potassium channel kv4.2, pT7:GST:linker:E4BP4 containing human E4BP4 and pcDNA3-rat-calcium receptor.

Primers and probe sets were selected as follows:

Plasmid HIV integrase
Forward PCR primer 5' - CAT TAG GAA GCA GCC CAG TAG TA - 3' (SEQ ID
NO:60)
Reverse PCR primer 5' - GGA TTT CTG CTG TCC CTG TAA TA - 3' (SEQ ID NO:61)
PCR amplicon size 1055 bp
T7 RNA transcript size 764 nt
Protection probe 5' - CTT AAC CGT AGC ACT GGT GAA ATT GCT GCC ATT GTC
TGT - 3' (SEQ ID NO:62)
Bridge probe 5' - GAC AAT GGC AGC AAT TTC ACC AGT GCT ACG GTT AAG
GTA AAT CTC CAG CGG AAG GGT ACG G - 3' (SEQ ID NO:63) (ZipCode sequence
is underlined.)

Plasmid GFP
Forward PCR primer 5' - GTG GGA GGT CTA TAT AAG CAG AGC - 3' (SEQ ID
NO:64)
Reverse PCR primer 5' - GGT ACC GTC GAC TGC AGA AT - 3' (SEQ ID NO:65)
PCR amplicon size 875 bp
T7 RNA transcript size 789 nt

Protection probe 5' - GTT CTT CTG CTT GTC GGC CAT GAT ATA GAC GTT
GTG - 3' (SEQ ID NO:66)
Bridge probe 5' - AAC GTC TAT ATC ATG GCC GAC AAG CAG AAG AAC
CTT TTC CCG TCC GTC ATC GCT CAA G - 3' (SEQ ID NO: 67) (ZipCode sequence is
underlined.)

Plasmid E4BP4
Forward PCR primer 5' - AGC AGC CCA GTA GTA GGT TGA G - 3' (SEQ ID NO:68)
Reverse PCR primer 5' - GGA GTC TTC TGA CAC TTC CGT TA - 3' (SEQ ID NO:69)
PCR amplicon size 1179 bp
T7 RNA transcript size 896 nt
Protection probe 5 '- CAA TTC TCG AAA CAC CGT ATC TAA TAT CCA AAA
CCG CTC CTT C - 3' (SEQ ID NO:70)
Bridge probe 5' - GGA GCG GTT TTG GAT ATT AGA TAC GGT GTT TCG AGA
ATT GCC GGC TTT GAA CTG CTC ACC GAT CT - 3' (SEQ ID NO:71) (ZipCode
sequence is underlined.)

Plasmid kv4.2
Forward PCR primer 5' - GGA CTT TCC TAC TTG GCA GTA CA - 3' (SEQ ID NO:72)
Reverse PCR primer 5' - CAA CCG TAA AGA TCA TGA CAC AG - 3' (SEQ ID NO:73
PCR amplicon size 1082 bp
T7 RNA transcript size 765 nt
Protection probe 5' - TGG GTA TCG TTC CAG GGT GTC TTG CCA TGT CTG - 3'
(SEQ ID NO:74)
Bridge probe 5' - ACA TGG CAA GAC ACC CTG GAA CGA TAC CCA CGA TCC
AAC GCA CTG GCC AAA CCT A - 3' (SEQ ID NO :75) (ZipCode sequence is
underlined.)

Plasmid Ca rec
Forward PCR primer 5' - GTG GGA GGT CTA TAT AAG CAG AGC - 3' (SEQ ID
NO:76)

Reverse PCR primer   5' - CCC GAC TAC ATG GAA ATA CTC AG - 3' (SEQ ID
NO:77)
PCR amplicon size     1050 bp
T7 RNA transcript size          964 nt
Protection probe 5' - CTA ATG CAC TCC ACA GAC TCT GGT CTT GAC TTC AGA
TC - 3'(SEQ ID NO:78)
Bridge probe   5' - CTG AAG TCA AGA CCA GAG TCT GTG GAG TGC ATT AGC
ACC ACC AGT GCC GCT ACC ACA ACG - 3' (SEQ ID NO:79) (ZipCode sequence is
underlined.)

PCR amplification of template and T7 transcription reaction was performed to generate control RNAs. PCR was performed on all five plasmid templates to generate amplicons containing the T7 RNA polymerase promoter and a portion of downstream gene sequence. PCR reactions were performed in 90 µl volumes containing 9 µl 10X Taq Gold Buffer I, 3.6 µl 10 mM dNTP mix, 1.8 µl 10 µM forward primer, 1.8 µl 10 µM reverse primer, 4 µl supercoiled plasmid (40 ng total), 0.9 µl Taq Gold (4.5 U) and 68.9 µl water. Cycling conditions were 95° C for 10 min, followed by 40 cycles of denaturation at 94°C for 30 sec, annealing at 60° C for 30 sec and extension at 72° C for 30 sec, ending with a 5 min incubation at 72° C and subsequent 4° C hold. The resulting DNA templates were transcribed using the Ambion Megascript kit with 60 µl reaction volumes and 2.5 hr reaction times. RNA yields for integrase, GFP and E4BP4 varied between 120 and 300µg. The kv4.2 and calcium receptor plasmids did not PCR amplify effectively and consequently produced much lower yields of RNA.

S 1 nuclease protection assay was performed , followed by S digestion and product hybridization to microspheres. Assays were first run without addition of RNA or S1 nuclease in order to validate the newly designed and synthesized bridge probes and protection probes for each of the five potential control targets (integrase, GFP, E4BP4, kv4.2 and Ca receptor). All five sets of probes gave strong signals (3000 - 10,000 median fluorescence intensity).

Throughout this application, various publications are referenced. The disclosures of these publications in their entireties are hereby incorporated by reference into this application in order to more fully describe the state of the art to which this invention pertains.

It will be apparent to those skilled in the art that various modifications and variations can be made in the present invention without departing from the scope or spirit of the invention. Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

## Claims

1. A method of identifying target nucleic acids in an array of assay samples, comprising
a. performing an arrayed nuclease protection assay to produce one or more assay products in each sample, wherein each assay product is specific for a target nucleic acid, wherein each assay product comprises a series of hybridized nucleic acid sequences, wherein one of the nucleic acid sequences is detectably tagged and wherein one of the nucleic acid sequences is attached to a distinguishably labeled microsphere; and
b. detecting with a detecting means the detectable tag and the distinguishable label in the assay products of each sample to identify the presence of a specific assay product,
the presence of specific assay products in the specific arrayed samples identifying the target nucleic acids.

2. A method of identifying target nucleic acids in an array of assay samples, comprising the steps of
a. contacting one or more protection probes with one or more target nucleic acids in the array of samples under conditions that allow protection probes complementary to the target nucleic acids to hybridize with the target nucleic acids to form first hybridization products, wherein each first hybridization product comprises a protection probe and a target nucleic acid, and wherein each protection probe comprises a detectable tag and a nucleic acid sequence complementary to one target nucleic acid to be identified in one or more specific samples in the array;
b. contacting the arrayed samples with a nuclease that cleaves non-hybridized nucleic acids, under conditions that allow the first hybridization products to be protected from the cleavage;
c. contacting the protection probes of the first hybridization products with one or more linker probes and one or more anchor probes under conditions that allow formation of one or more second hybridization products, wherein the second hybridization products comprise a protection probe, a linker probe, and an anchor probe, wherein each linker probe comprises a sequence that is capable of hybridizing with one protection probe and a sequence that is capable of hybridizing with one anchor probe in the sample, and wherein each anchor probe is coupled with a microsphere with a distinguishable label; and
d. detecting the presence of the detectable tag of the protection probe and the label of the microsphere in each second hybridization product in each sample;
the presence of the detectable tag and the label of the microsphere in a specific sample in the array identifying the target nucleic acids.

3. The method of claim 1 or 2, further comprising quantification of the identified target nucleic acids, wherein the amount of detectable tag associated with each distinguishable label in the specific arrayed samples quantifying the target nucleic acids.

4. The method of claims 1 or 3, wherein the assay product comprises a detectably labeled protection probe.

5. The method of claims 1 or 3, wherein each assay product comprises a series of three or four hybridized nucleic acid sequences.

6. The method of claim 2, wherein the protection probes are less than 75 bases in length.

7. The method of claim 2, wherein the linker probe further comprises a spacer sequence between the sequence that is capable of hybridizing with the protection probe and the sequence capable of hybridizing with the anchor probe.

8. The method of claim 2, wherein each sample in the array contains a different set of protection probes.

9. The method of claim 2, wherein the same set of anchor probes coupled with the same set of microspheres is present in each sample of the array.

10. The method of claims 1, 2, or 3, wherein the distinguishable labels of the microspheres are fluorescence labels.

11. The method of claim 1, 2, or 3, wherein the same set of distinguishably labeled microspheres is present in each sample of the array.

12. The method of claim 3, wherein each second hybridization product comprises three or four hybridized nucleic acid sequences which include a protection probe, a linker probe, and an anchor probe.

13. The method of claims 1, 2, or 3, wherein each assay sample contains one or more internal controls.

14. A kit for identifying or quantifying target nucleic acids in an array of assay samples, comprising a set of microspheres coupled with a set of anchor probes, wherein the set of microspheres comprises subsets of microspheres having distinguishable labels, wherein the set of anchor probes comprises a subset of probes, wherein each subset of anchor probes is coupled with only one subset of microspheres, and wherein each subset of anchor probes is capable of hybridizing under hybridizing conditions to a subset of linker probes specific for a target nucleic acid to be identified or quantified.

15. The kit of claim 14, further comprising one or more internal controls for each assay sample.


SEQUENCE LISTING

<110>

      Chen, Jingwen
      Taylor, David D.
      Weiner, Michael P.
      Ye, Fei

<120> MULTIPLEXED GENE ANALYSIS ON A MOBILE
  SOLID SUPPORT

<130> 07083.0011P1

<150> 09/834,470
<151> 2001-04-13

<160> 79

<170> FastSEQ for Windows Version 4.0

<210> 1
<211> 25
<212> DNA
<213> Artificial Sequence

<220>
<223> Description of Artificial Sequence:/note =
      synthetic construct

<400> 1
gatgatcgac gagacactct cgcca
   25

<210> 2
<211> 25
<212> DNA
<213> Artificial Sequence

<220>
<223> Description of Artificial Sequence:/note =
      synthetic construct

<400> 2
cggtcgacga gctgccgcgc aagat
   25

<210> 3
<211> 25
<212> DNA
<213> Artificial Sequence

<220>
<223> Description of Artificial Sequence:/note =
      synthetic construct

<400> 3
gacattcgcg atcgccgccc gcttt
   25

<210> 4
<211> 25
<212> DNA
<213> Artificial Sequence

<220>
<223> Description of Artificial Sequence:/note =
      synthetic construct

<400> 4
cggtatcgcg accgcatccc aatct
   25

<210> 5
<211> 25
<212> DNA
<213> Artificial Sequence

<220>
<223> Description of Artificial Sequence:/note =
      synthetic construct

<400> 5
gctcgaagag gcgctacaga tcctc
   25

<210> 6
<211> 25
<212> DNA
<213> Artificial Sequence

<220>
<223> Description of Artificial Sequence:/note =
      synthetic construct

<400> 6
caccgccagc tcggcttcga gttcg
   25

<210> 7
<211> 25
<212> DNA
<213> Artificial Sequence

<220>
<223> Description of Artificial Sequence:/note =
      synthetic construct

<400> 7
cgactccctg tttgtgatgg accac
   25

<210> 8

<211> 25
<212> DNA
<213> Artificial Sequence

<220>
<223> Description of Artificial Sequence:/note =
      synthetic construct

<400> 8
cttttcccgt ccgtcatcgc tcaag
   25

<210> 9
<211> 25
<212> DNA
<213> Artificial Sequence

<220>
<223> Description of Artificial Sequence:/note =
      synthetic construct

<400> 9
ggctgggtct acagatcccc aactt
   25

<210> 10
<211> 25
<212> DNA
<213> Artificial Sequence

<220>
<223> Description of Artificial Sequence:/note =
      synthetic construct

<400> 10
gaacctttcg cttcaccggc cgatc
   25

<210> 11
<211> 25
<212> DNA
<213> Artificial Sequence

<220>
<223> Description of Artificial Sequence:/note =
      synthetic construct

<400> 11
tttcggcacg cgcgggatca ccatc
   25

<210> 12
<211> 25
<212> DNA
<213> Artificial Sequence

<220>

<223> Description of Artificial Sequence:/note =
      synthetic construct

<400> 12
ctcggtggtg ctgacggtgc aatcc
   25

<210> 13
<211> 25
<212> DNA
<213> Artificial Sequence

<220>
<223> Description of Artificial Sequence:/note =
      synthetic construct

<400> 13
tcaacgtgcc agcgccgtcc tggga
   25

<210> 14
<211> 25
<212> DNA
<213> Artificial Sequence

<220>
<223> Description of Artificial Sequence:/note =
      synthetic construct

<400> 14
gcgaaggaac tcgacgtgga cgccg
   25

<210> 15
<211> 25
<212> DNA
<213> Artificial Sequence

<220>
<223> Description of Artificial Sequence:/note =
      synthetic construct

<400> 15
cggggatacc gatctcgggc gcaca
   25

<210> 16
<211> 25
<212> DNA
<213> Artificial Sequence

<220>
<223> Description of Artificial Sequence:/note =
      synthetic construct

<400> 16
ggagcttacg ccatcacgat gcgat

25

<210> 17
<211> 25
<212> DNA
<213> Artificial Sequence

<220>
<223> Description of Artificial Sequence:/note =
      synthetic construct

<400> 17
cgtggcggtg cggagtttcc ccgaa
   25

<210> 18
<211> 25
<212> DNA
<213> Artificial Sequence

<220>
<223> Description of Artificial Sequence:/note =
      synthetic construct

<400> 18
cgatccaacg cactggccaa accta
   25

<210> 19
<211> 25
<212> DNA
<213> Artificial Sequence

<220>
<223> Description of Artificial Sequence:/note =
      synthetic construct

<400> 19
ctgaatcctc caaccgggtt gtcga
   25

<210> 20
<211> 25
<212> DNA
<213> Artificial Sequence

<220>
<223> Description of Artificial Sequence:/note =
      synthetic construct

<400> 20
ttcggcgctg gcgtaaagct tttgg
   25

<210> 21
<211> 25
<212> DNA

<213> Artificial Sequence

<220>
<223> Description of Artificial Sequence:/note =
      synthetic construct

<400> 21
gtaaatctcc agcggaaggg tacgg
   25

<210> 22
<211> 25
<212> DNA
<213> Artificial Sequence

<220>
<223> Description of Artificial Sequence:/note =
      synthetic construct

<400> 22
ccggctttga actgctcacc gatct
   25

<210> 23
<211> 25
<212> DNA
<213> Artificial Sequence

<220>
<223> Description of Artificial Sequence:/note =
      synthetic construct

<400> 23
actacgcaac accgaacgga taccc
   25

<210> 24
<211> 25
<212> DNA
<213> Artificial Sequence

<220>
<223> Description of Artificial Sequence:/note =
      synthetic construct

<400> 24
ggaccaatgg tcccattgac caggt
   25

<210> 25
<211> 25
<212> DNA
<213> Artificial Sequence

<220>
<223> Description of Artificial Sequence:/note =
      synthetic construct

<400> 25
caacgctgag cgcgtcactg acata
   25

<210> 26
<211> 25
<212> DNA
<213> Artificial Sequence

<220>
<223> Description of Artificial Sequence:/note =
      synthetic construct

<400> 26
gagacaaagg tctgcgccag cacca
   25

<210> 27
<211> 25
<212> DNA
<213> Artificial Sequence

<220>
<223> Description of Artificial Sequence:/note =
      synthetic construct

<400> 27
tggccacact gtccatttgc gcggt
   25

<210> 28
<211> 25
<212> DNA
<213> Artificial Sequence

<220>
<223> Description of Artificial Sequence:/note =
      synthetic construct

<400> 28
   ccttgcgacg tgtcaagttg gggtc
      25

<210> 29
<211> 25
<212> DNA
<213> Artificial Sequence

<220>
<223> Description of Artificial Sequence:/note =
      synthetic construct

<400> 29
aggttagggt cgcgccaaac tctcc
   25

<210> 30
<211> 25
<212> DNA
<213> Artificial Sequence

<220>
<223> Description of Artificial Sequence:/note =
      synthetic construct

<400> 30
acgactgcga ggtgcggtaa gcaca
   25

<210> 31
<211> 25
<212> DNA
<213> Artificial Sequence

<220>
<223> Description of Artificial Sequence:/note =
      synthetic construct

<400> 31
gcgatcgccg ggagatatac ccaac
   25

<210> 32
<211> 25
<212> DNA
<213> Artificial Sequence

<220>
<223> Description of Artificial Sequence:/note =
      synthetic construct

<400> 32
tcgtgccgga ctcgagcacc aatac
   25

<210> 33
<211> 25
<212> DNA
<213> Artificial Sequence

<220>
<223> Description of Artificial Sequence:/note =
      synthetic construct

<400> 33
gctttagcac cgcgatggcg tagac
   25

<210> 34
<211> 25
<212> DNA
<213> Artificial Sequence

<220>
<223> Description of Artificial Sequence:/note =
      synthetic construct

<400> 34
cagccgcggt actgaatgcg atgct
   25

<210> 35
<211> 25
<212> DNA
<213> Artificial Sequence

<220>
<223> Description of Artificial Sequence:/note =
      synthetic construct

<400> 35
ccccggatag ctgacgaggc ttacg
   25

<210> 36
<211> 25
<212> DNA
<213> Artificial Sequence

<220>
<223> Description of Artificial Sequence:/note =
      synthetic construct

<400> 36
tccggacagg ttggggtgcg tttgg
   25

<210> 37
<211> 25
<212> DNA
<213> Artificial Sequence

<220>
<223> Description of Artificial Sequence:/note =
      synthetic construct

<400> 37
cgtagagcaa cgcgataccc ccgac
   25

<210> 38
<211> 25
<212> DNA
<213> Artificial Sequence

<220>
<223> Description of Artificial Sequence:/note =
      synthetic construct

<400> 38

agcagcagtg acaatgccac cgccg
   25

<210> 39
<211> 25
<212> DNA
<213> Artificial Sequence

<220>
<223> Description of Artificial Sequence:/note =
      synthetic construct

<400> 39
tcgcccgcgg acaccgagaa ttcga
   25

<210> 40
<211> 25
<212> DNA
<213> Artificial Sequence

<220>
<223> Description of Artificial Sequence:/note =
      synthetic construct

<400> 40
gaggcagatc cgtaggcggg tgcat
   25

<210> 41
<211> 25
<212> DNA
<213> Artificial Sequence

<220>
<223> Description of Artificial Sequence:/note =
      synthetic construct

<400> 41
gcgatagcca gtgccgccaa tcgtc
   25

<210> 42
<211> 25
<212> DNA
<213> Artificial Sequence
<220>
<223> Description of Artificial Sequence:/note =
      synthetic construct

<400> 42
agcggtcacc atggccacga actgc
   25

<210> 43
<211> 25
<212> DNA

<213> Artificial Sequence

<220>
<223> Description of Artificial Sequence:/note =
      synthetic construct

<400> 43
ttgcaacagc agcccgactc gacgg
   25

<210> 44
<211> 25
<212> DNA
<213> Artificial Sequence

<220>
<223> Description of Artificial Sequence:/note =
      synthetic construct

<400> 44
tgactccggc gatacgggct ccgaa
   25

<210> 45
<211> 25
<212> DNA
<213> Artificial Sequence

<220>
<223> Description of Artificial Sequence:/note =
      synthetic construct

<400> 45
accggctacc tggtatcggt cccga
   25

<210> 46
<211> 25
<212> DNA
<213> Artificial Sequence

<220>
<223> Description of Artificial Sequence:/note =
      synthetic construct

<400> 46
gagcgagcgg gcaaacgcca gtact
   25

<210> 47
<211> 25
<212> DNA
<213> Artificial Sequence

<220>
<223> Description of Artificial Sequence:/note =
      synthetic construct

<400> 47
agtcgaagtg ggcggcgtca gactc
   25

<210> 48
<211> 25
<212> DNA
<213> Artificial Sequence

<220>
<223> Description of Artificial Sequence:/note =
      synthetic construct

<400> 48
caccaccagt gccgctacca caacg
   25

<210> 49
<211> 25
<212> DNA
<213> Artificial Sequence

<220>
<223> Description of Artificial Sequence:/note =
      synthetic construct

<400> 49
ccgtgttaac ggcgcgacgc aagga
   25

<210> 50
<211> 25
<212> DNA
<213> Artificial Sequence

<220>
<223> Description of Artificial Sequence:/note =
      synthetic construct

<400> 50
gagtgaacgc agactgcagc gaggc
   25

<210> 51
<211> 25
<212> DNA
<213> Artificial Sequence

<220>
<223> Description of Artificial Sequence:/note =
      synthetic construct

<400> 51
cggcggtctt cacgctcaac agcag
   25

<210> 52
<211> 25
<212> DNA
<213> Artificial Sequence

<220>
<223> Description of Artificial Sequence:/note =
      synthetic construct

<400> 52
gttgggcccg agcactgcaa gcacc
   25

<210> 53
<211> 25
<212> DNA
<213> Artificial Sequence

<220>
<223> Description of Artificial Sequence:/note =
      synthetic construct

<400> 53
tcggcgtacg agcacccaca cccag
   25

<210> 54
<211> 25
<212> DNA
<213> Artificial Sequence

<220>
<223> Description of Artificial Sequence:/note =
      synthetic construct

<400> 54
ccccaaacgt accaagcccg cgtcg
   25

<210> 55
<211> 25
<212> DNA
<213> Artificial Sequence
<220>
<223> Description of Artificial Sequence:/note =
      synthetic construct

<400> 55
atggcaccga cggctggcag accac
   25

<210> 56
<211> 25
<212> DNA
<213> Artificial Sequence

<220>

<223> Description of Artificial Sequence:/note =
      synthetic construct

<400> 56
agccgcgaac accacgatcg accgg
   25

<210> 57
<211> 25
<212> DNA
<213> Artificial Sequence

<220>
<223> Description of Artificial Sequence:/note =
      synthetic construct

<400> 57
cgcgcgcagc tgcagcttgc tcatg
   25

<210> 58
<211> 25
<212> DNA
<213> Artificial Sequence

<220>
<223> Description of Artificial Sequence:/note =
      synthetic construct

<400> 58
taccggcggc agcaccagcg gtaac
   25

<210> 59
<211> 20
<212> DNA
<213> Artificial Sequence

<220>
<223> Description of Artificial Sequence:/note =
      synthetic construct

<400> 59
caggccaagt aacttcttcg
   20

<210> 60
<211> 23
<212> DNA
<213> Artificial Sequence

<220>
<223> Description of Artificial Sequence:/note =
      synthetic construct

<400> 60
cattaggaag cagcccagta gta

23

<210> 61
<211> 23
<212> DNA
<213> Artificial Sequence

<220>
<223> Description of Artificial Sequence:/note =
      synthetic construct

<400> 61
ggatttctgc tgtccctgta ata
   23

<210> 62
<211> 39
<212> DNA
<213> Artificial Sequence

<220>
<223> Description of Artificial Sequence:/note =
      synthetic construct

<400> 62
cttaaccgta gcactggtga aattgctgcc attgtctgt
   39

<210> 63
<211> 61
<212> DNA
<213> Artificial Sequence

<220>
<223> Description of Artificial Sequence:/note =
      synthetic construct

<400> 63
gacaatggca gcaatttcac cagtgctacg gttaaggtaa atctccagcg gaagggtacg
   60
g
   61

<210> 64
<211> 24
<212> DNA
<213> Artificial Sequence

<220>
<223> Description of Artificial Sequence:/note =
      synthetic construct

<400> 64
gtgggaggtc tatataagca gagc
   24

<210> 65

<211> 20
<212> DNA
<213> Artificial Sequence

<220>
<223> Description of Artificial Sequence:/note =
      synthetic construct

<400> 65
ggtaccgtcg actgcagaat
   20

<210> 66
<211> 36
<212> DNA
<213> Artificial Sequence

<220>
<223> Description of Artificial Sequence:/note =
      synthetic construct

<400> 66
gttcttctgc ttgtcggcca tgatatagac gttgtg
   36

<210> 67
<211> 58
<212> DNA
<213> Artificial Sequence

<220>
<223> Description of Artificial Sequence:/note =
      synthetic construct

<400> 67
aacgtctata tcatggccga caagcagaag aaccttttcc cgtccgtcat cgctcaag
   58

<210> 68
<211> 22
<212> DNA
<213> Artificial Sequence

<220>
<223> Description of Artificial Sequence:/note =
      synthetic construct

<400> 68
agcagcccag tagtaggttg ag
   22

<210> 69
<211> 23
<212> DNA
<213> Artificial Sequence

<220>

<223> Description of Artificial Sequence:/note =
      synthetic construct

<400> 69
ggagtcttct gacacttccg tta
   23

<210> 70
<211> 43
<212> DNA
<213> Artificial Sequence

<220>
<223> Description of Artificial Sequence:/note =
      synthetic construct

<400> 70
caattctcga aacaccgtat ctaatatcca aaaccgctcc ttc
   43

<210> 71
<211> 65
<212> DNA
<213> Artificial Sequence

<220>
<223> Description of Artificial Sequence:/note =
      synthetic construct

<400> 71
ggagcggttt tggatattag atacggtgtt tcgagaattg ccggctttga actgctcacc
   60
gatct
   65

<210> 72
<211> 23
<212> DNA
<213> Artificial Sequence
<220>
<223> Description of Artificial Sequence:/note =
      synthetic construct

<400> 72
ggactttcct acttggcagt aca
   23

<210> 73
<211> 23
<212> DNA
<213> Artificial Sequence

<220>
<223> Description of Artificial Sequence:/note =
      synthetic construct

<400> 73

caaccgtaaa gatcatgaca cag
   23

<210> 74
<211> 33
<212> DNA
<213> Artificial Sequence

<220>
<223> Description of Artificial Sequence:/note =
      synthetic construct

<400> 74
tgggtatcgt tccagggtgt cttgccatgt ctg
   33

<210> 75
<211> 55
<212> DNA
<213> Artificial Sequence

<220>
<223> Description of Artificial Sequence:/note =
      synthetic construct

<400> 75
acatggcaag acaccctgga acgataccca cgatccaacg cactggccaa accta
   55

<210> 76
<211> 24
<212> DNA
<213> Artificial Sequence

<220>
<223> Description of Artificial Sequence:/note =
      synthetic construct

<400> 76
gtgggaggtc tatataagca gagc
   24

<210> 77
<211> 23
<212> DNA
<213> Artificial Sequence

<220>
<223> Description of Artificial Sequence:/note =
      synthetic construct

<400> 77
cccgactaca tggaaatact cag
   23

<210> 78
<211> 38

<212> DNA
<213> Artificial Sequence

<220>
<223> Description of Artificial Sequence:/note =
      synthetic construct

<400> 78
ctaatgcact ccacagactc tggtcttgac ttcagatc
   38

<210> 79
<211> 60
<212> DNA
<213> Artificial Sequence

<220>
<223> Description of Artificial Sequence:/note =
      synthetic construct

<400> 79
ctgaagtcaa gaccagagtc tgtggagtgc attagcacca ccagtgccgc taccacaacg
   60